# EUROPEAN PATENT SPECIFICATION

(11) **EP 2 022 771 B1**
(45) Date of publication and mention of the grant of the patent: **16.03.2011**
(21) Application number: 06743465.4
(22) Date of filing: 08.05.2006
(51) Int. Cl.: C07C 2/66, C07C 15/107, C07C 7/05, C07C 7/13, B01D 15/00, C11D 3/18

(54) **METHOD FOR PURIFYING ALKYL AROMATIC COMPOUNDS**
VERFAHREN ZUR AUFREINIGUNG VON AROMATISCHEN ALKYLVERBINDUNGEN
PROCÉDÉ DE PURIFICATION DE COMPOSÉS ALKYLAROMATIQUES

(43) Date of publication of application: 11.02.2009
(73) Proprietor: CEPSA QUIMICA , S.A., 28042 Madrid (ES)
(72) Inventor: GONCALVES ALMEIDA, José Luis, 11204 Algeci Ras, Cádiz (ES); BERNATEJERO, José Luis, 28066 Boadilla Del Monte, Madrid (ES)
(74) Representative: Carvajal y Urquijo, Isabel
(86) International application number: PCT/ES2006/000217
(87) International publication number: WO 2007/128841

(56) References cited:
- EP-A2- 0 520 975
- US-A- 4 433 196
- US-A- 4 691 068
- US-A- 5 157 158
- US-A- 6 069 285
- US-B1- 6 169 219

## Description

### Field of the Invention

The present invention relates generally to purification processes and specifically to the purification of alkyl aromatic compounds by means of clay-based treaters.

### State of the Art

Alkyl aromatic compounds are an important family of substances that are used as raw materials in many industrial fields, such as the field of plasticizers, polymeric materials, insecticides, in agriculture for preventing the agglomeration of fertilizers, in the manufacture of textiles and fabrics, in the leather and fur industry, herbicides, industrial cleaning processes, in the photography industry, in the manufacture of adhesives and in fire-fighting products such as wetting agents, in electrochemical processes for removing dirt and grease from the surface of a substrate, and in biodegradable detergents (Surfactants in Consumers Products, Theory, Technology, and Application, Edited by J. Falbe, Springer Verlag, 1987).

In the event of using alkyl aromatic compounds within the field of detergents, once sulfonated and neutralized, one of the parameters considered when analyzing the quality of the product is its color. Although said parameter has no influence on its detersive properties, color has a considerable economic relevance especially when alkyl benzene sulfonates are used in liquid formulations. When a liquid or gel detergent is produced with a high content of active ingredient, the chromophore species present in the alkyl benzene sulfonate affect the color of the product, conferring it a yellowish brown tonality masking the color of the coloring additives. This forces using larger amounts of coloring additives to achieve a certain color, and even in these cases, some color ranges such as light blue cannot be achieved. There is furthermore another problem related to the presence of these chromophore species in alkyl benzene sulfonates. Even when these chromophore species appear at a trace level, they can inhibit the sulfonability of the alkyl benzene about 1% by weight, thus decreasing the efficiency of the process. By means of this invention, the amount of chromophore by-products associated to HF alkylation technology is minimized, the efficiency of the process and the final quality of the product thus increasing.

To evaluate the origin of the chromophore species decreasing the quality of linear alkyl aromatic compounds, it is necessary to analyze the process used to produce said compounds. The integrated process for producing linear alkyl benzene (LAB) is described in the Handbook of Petroleum Refining Process, edited by Robert A. Meyers, 1986, pp.1-23. The usual process used by the petrochemical industry to produce alkyl aromatic compounds, especially for detergent applications, consists of dehydrogenating linear paraffins to obtain the corresponding olefins, which are later used to alkylate benzene. Patent U.S. 5,276,231, describes the intermediate steps in the LAB production, such as the selective hydrogenation of diolefin by-products to monoolefins and the elimination of non-linear by-products, both of them contained in the effluent of the dehydrogenation step. Alkyl aromatic products are commercially called linear alkyl benzene-LAB. Linear alkyl benzene sulfonate-LAS is produced by sulfonating linear alkyl benzene-LAB followed by neutralizing the sulfonic acid (HLAS) produced. The linear olefins used in the alkylation process have between nine and sixteen carbon atoms. The alkylation step occurs in liquid phase, in the presence of a Friedel-Crafts type catalyst such as AlCl₃ or hydrofluoric acid (HF), for example. The process using HF is well known and used on a commercial level, providing a high yield of LAB (greater than 99% by weight) with a selectivity for 2-phenyl isomers less than 20% by weight of LAB. Solid catalysts are also used for this alkylation process. The state of the art discloses the use of a number of solid acid catalysts for producing phenyl alkanes, such as synthetic such as synthetic faujasites (zeolites X and Y), zeolite L, ZSM-5, ZSM-18, ZSM-20, mordenite and offretite.

The alkyl benzenes produced by means of HF technology in the detergent range (C₁₀-phenyl to C₁₄-phenyl) are colorless. The color appears when the LAB is sulfonated to obtain the corresponding sulfonic acids. This means that the compounds that effectively generate color are formed in this step; however it is commonly accepted that chromophore precursors are generated in previous steps of the LAB production process. Molecules having a polyaromatic structure, in which the presence of a number of conjugated double bonds allows easy electron transits, are considered to be potential chromophore precursors. This fact is also related to the inhibition in the sulfonability of the alkyl benzene when it contains said chromophore precursors. As the sulfonation occurs through a carbocation type reaction in the aromatic ring, polyaromatic compounds can be sulfonated more easily than alkyl benzenes due to the fact that the polyaromatic structure can support a larger amount of resonant intermediate species and in a more stabilized manner than a single aromatic ring.

Based on the previous considerations, there are two steps in the LAB production process in which the chromophore precursors can be generated. The first step is the paraffin dehydrogenation step in order to obtain olefins. In this step, consecutive dehydrogenation reactions combined with diffusion limitations can lead to the formation of alkyl aromatic compounds such as alkylindanes, and also of short-chain alkyl polyaromatic compounds such as alkylnaphthalenes, phenanthrenes, anthracenes and fluorenes. The olefins produced can also undergo oligomerization processes. These compounds are partially removed from the process during the HF regeneration step, in which purified HF is obtained through the distillation column head stream and the oligomers and other by-products are removed through the bottom stream. Furthermore, the alkyl aromatic compounds present in the paraffin recirculation can be dehydrogenated, generating new aromatic species.

Platinum, the typical active metal present in catalysts used in dehydrogenation processes, is one of the catalysts that can give rise to said reactions. The temperatures required for aromatization reactions to occur are comprised between 400 and 500°C, which is the range in which normal paraffins are dehydrogenated. The commonly proposed mechanism for dehydrocyclization (E. F. G. Herington and E. K. Riedel, Proc. Roy. Soc. London, Ser. A, 184, 434-447, (1945); H. Pines and C. T. Chen, J. Org. Chem., 26, 1057, (1961); H. Pines and C. T. Goetshel, J. Org. Chem., 30, 3530, (1965) and H. Pines, C. T. Goetshel and J. W. Dembinski, J. Org. Chem., 30, 3540, (1965)) suggests that all the possible monoolefins are formed in a first step. These monoolefins can generate intermediate α,β-diadsorbed species in the active centers of the catalyst, which can undergo a quick dehydrogenation resulting in the formation of benzene derivatives (through a carbon1-carbon6 type closure). According to this proposal, 1-heptene and 2-heptene are transformed into toluene in a quick reaction. However, 3-heptene reacts slowly due to the fact that a previous migration of the double bond is necessary for forming the six-member aromatic ring.

The aromatization of n-octane yields ethylbenzene and ortho-xylene, the products of the direct 1-6 closure, but meta- and para-xylenes are also generated. Heavier paraffins produce the corresponding alkyl benzenes. This means that, in the event of initially using C₁₀-C₁₄ paraffins, C₄-phenyl to C₈-phenyl type compounds as well as short-chain dialkyl aromatic species could be formed. The possible sequential dehydrogenation of the initial paraffin and of the resulting olefins, with the formation of conjugated trienes which can affect the formation of aromatic compounds must also be taken into account, as it considered by F. M. Dautzemberg and J. L. Platteeuw, J. Catal., 19, 41, (1970) and Z. Paál and P. Tétényi, J. Catal., 30, 350, (1973).

In addition to the sequential dehydrogenation on metal centers, the formation of rings from normal paraffins can also occur through the carbocation processes derived from an initial paraffin dehydrogenation step on the acid centers of the alumina used as the typical support of the active metal. This reaction could be developed on an adjacent acid-base pair, as considered by B. C. Gates, J. R. Katzer, G. C. A. Schuit, Chemistry of catalytic process, McGraw-Hill Book Company, page 277, 1st edition, (1979).

The active metal can have an important role in the reactions via carbocation generated in the acid centers of the alumina since it can generate carbocations from existing olefins, as considered by G. A. Olah and A. Molnár, Hydrocarbon chemistry, John Wiley &Sons Inc., 2nd Edition, page 54, (2003).

In addition, alkyl aromatic compounds having a sufficiently long side chain can also undergo dehydrocyclization processes. In fact, the aromatic ring enhances this reaction, since alkyl aromatic compounds undergo dehydrocyclization processes much more quickly than paraffins. Therefore, isolated or condensed rings can be formed depending on the length of the side chain. Thus, on platinum active centers, n-butylbenzene generates methylindane and naphthalene, whereas n-pentylbenzene produces methylnaphthalene as the main product, as considered by S. M. Csicsery, J. Cat., 15, 111, (1969).

All the previously mentioned transformations can explain the formation of alkyl aromatic, polyaromatic and alkyl or polyalkyl aromatic compounds, potential chromophore precursors, in the dehydrogenation step.

It must be emphasized that, apart from the aromatic compound which is fed to the alkylation step, any previously considered aromatic or polyaromatic compound can undergo additional alkylation processes, producing alkyl or polyalkyl aromatic compounds.

Cyclization reactions can also occur in the alkylation step with HF when there are diolefins in the medium. Diolefins can react with aromatic and polyaromatic compounds by means of a Diels Alder addition type cyclization, generating dialkylindane and/or dialkyltetralin type compounds.

In any case, with the development of the selective dehydrogenation step, the formation of by-products derived from diolefin species in alkylation processes with HF is reduced to a great extent as a result of the elimination of said diolefins. The typical products and by-products of the alkylation process with HF are classified in a practical manner according to the following classification:
*Alkylate: They are monoalkyl benzenes. This group comprises compounds from C₁₀-phenyl to C₁₃-phenyl, i.e. between 16 and 19 carbon atoms per molecule.
*Heavy Alkylate: This name includes all the compounds the molecular weight of which is greater than that of the heaviest monoalkylated compound produced. This means that the products classified here contain a number of carbon atoms equal to or greater than 20. Two subcategories can be defined within this family:
   > Dialkyl benzenes (DAB): This group comprises the products the total number of carbon atoms of which is comprised between 26 and 32. It is mostly formed by dialkyl benzenes. These products are generated by dialkylating an aromatic molecule with two olefin molecules. This group distinguishes from the lightest dialkyl benzenes (corresponding to didecylbenzene, containing 26 carbon atoms) to the heaviest ones (corresponding to ditridecylbenzene, with 32 carbon atoms). This group of by-products can be easily separated from the alkylate by means of a distillation process due to the difference between their distillation ranges.
   > Light dialkyl benzenes (L-DAB): This group comprises the by-products the total number of carbon atoms of which is comprised between 20 and 26. These compounds are formed by alkylating light alkylates produced in the dehydrogenation step with olefins of the C₁₀-C₁₃ range. Said group includes C₁₄-phenyl (which is generated when the initial paraffin feedstock contains C₁₄) and heavier compounds, such as diphenylalkanes, fluorenes, monoalkylated polyaromatic compounds, indanes, alkylphenyl indenes, tetralins, etc. This group of by-products cannot be perfectly separated from the alkylate by means of a distillation process because the distillation ranges are very close to one another. It must be taken into account that chromophore compounds have an unwanted effect at a trace level.
*Light compounds: These compounds have a molecular weight less than that of the lightest alkylate comprised within the alkylate group (C10-phenyl). This means that the products included here contain a number of carbon atoms less than or equal to 16. This group comprises short-chain alkyl aromatic compounds and also polyaromatic compounds and other by-products generated by cracking processes.
*Crude alkylate: This name refers to the mixture of alkylate and heavy alkylate coming from the alkylation step, once they have been separated from unreacted benzene, light compounds and paraffins.

The use of clay treaters is generally applied to aromatic species alkylation process when an acid solid is used as a catalyst. The state of the art shows the use of a number of solid acid catalysts for producing alkyl benzene, such as synthetic faujasites (zeolites X and Y), zeolite L, ZSM-5, ZSM-18, ZSM-20, mordenite and offretite, amorphous silica-alumina, etc. This type of catalytic process generates a similar amount of heavy by-products as HF technology. In the process with HF, part of the chromophore compounds formed in the previous steps and also in the alkylation step are transferred to the HF phase, thus being separated from crude alkylate, and are then removed from the process in the HF regeneration column. This acid washing step does not exist in the processes using solid catalysts, therefore said heavy by-products remain in the alkyl benzene stream. Therefore, the technology based on solid catalysts requires a subsequent purification step to clean the alkylate. Thus, patent US 5,157,158 discloses the use of a clay treater for the case of alkylation with solid catalysts. There are also other patents related to the elimination of color precursors in alkyl benzenes obtained by means of alkylation processes based on AlCl₃ technology, proposing the use of alumina-based purifying solids, such as bauxite clays (patent US 4,468,476) or bauxite clays deposited on alumina matrixes (patent US 4,433,196).

The typical configuration of a clay treater in an alkylation process based on solid catalysts is shown in patent US 5,157,158, in which it is described how all the "crude alkylate", stream 10; is fed to the distillation column, unit 101. The alkylate is separated through the head of the column, stream 20, whereas the heavy alkylate leaves the column through the bottom stream, stream 30. All the alkylate, stream 20, is fed to the clay treater, unit 201, and then, through stream 40, to the benzene stripper, unit 401. The benzene produced in the clay treater is separated from the alkylate, leaving the stripping column through its head, stream 50, and being recirculated to the process. After the stripping column, the purified alkylate is sent directly to the storage tank through stream 60, without returning to the process. The quality of the final alkylate is good because almost 100% of the initial heavy alkylate is separated in the initial distillation column, but the operating costs are high since the entire the alkylate stream has to be treated.

It is therefore necessary to manufacture detergents having a very low sulfonation color and the manufacturing process of which has very low associated economic costs.

### Description of the Invention

The present invention relates to a method for purifying alkyl aromatic compounds based on a system for percolating the fraction of the alkylate in which the color precursors are concentrated using a selected clay placed in a fixed-bed reactor. The impurities are eliminated in conditions minimizing secondary reactions, providing a final alkylate with minimum sulfonation color and with very low operating costs, such that the drawbacks set forth in the state of the art are solved.

Thus, a first aspect of the invention relates to a method for purifying alkyl aromatic compounds comprising the following steps:
i) separating in a distillation column a mixture of alkyl aromatic compounds to give rise to a light fraction relatively free from chromophore precursors and a heavy fraction in which the chromophore precursors are concentrated,
ii) separating in a distillation column the heavy fraction of step i) to give rise to a light fraction formed by alkylate with a certain amount of chromophore precursors and a heavy fraction mainly formed by heavy alkylate, said heavy alkylate is stored in tanks,
iii) eliminating the chromophore precursors from the light fraction of step ii) by means of fixed-bed percolation with a purifying solid, said purifying solid is a selected clay.
iv) Eliminating by means of a refining column the light by-products formed in step iii) such that a purified alkylate is obtained.
v) Mixing the purified alkylate obtained in step iv) with the lightest fraction obtained in the distillation of step i), such that an alkylate stream is generated which, once sulfonated, provides an alkyl benzene sulfonate with very low sulfonation color.

In a particular embodiment of the present invention, the alkyl aromatic compound to be purified comprises an alkyl chain with 9 to 25 carbon atoms.

In another particular embodiment, at least 85% by weight of the alkyl aromatic compounds comprise linear alkyl groups.

In a particular embodiment, the aromatic group forming the alkyl aromatic compounds is selected from the group consisting of: benzene, toluene and xylene.

In a particular embodiment, the alkyl aromatic compound comprises a maximum of 1% by weight of aromatic compounds.

In a particular embodiment, the alkyl aromatic compound comprises a maximum of 1% by weight of paraffins.

In a particular embodiment, the distillation column of step i) separates between 60-80% by weight of the feedstock through its head, it preferably separates 75% by weight of the feedstock through its head.

In a particular embodiment, the distillation column of step i) operates at a bottom temperature comprised between 150-250°C.

In a particular embodiment of the present invention, the distillation column of step i) operates at a head temperature comprised between 90-175°C.

In a particular embodiment of the present invention, the distillation column of step i) operates at a head pressure comprised between 0-1 bar.

In a particular embodiment of the present invention, the distillation column of step ii) operates at a bottom temperature comprised between 175-290°C.

In a particular embodiment of the present invention, the distillation column of step ii) operates at a head temperature comprised between 90-200°C.

In a particular embodiment of the present invention, the distillation column of step ii) operates at a head pressure comprised between 0-1 bar.

In a particular embodiment of the present invention, the chromophore precursor elimination step iii) is carried out at a temperature comprised between 50-150°C, preferably between 60-140 °C.

In a particular embodiment of the present invention, the chromophore precursor elimination step iii) is carried out at a liquid space velocity comprised between 0.3-4 h⁻¹.

In a particular embodiment of the present invention, the purifying solid of step iii) is selected from the group consisting of: zeolite, molecular sieve, silica, silica-alumina, silica gel, macroporous magnesium silicate, activated alumina, modified smectite, cellulose acetate, macroporous polystyrene gel, activated carbon and high performance organoselective polymeric membranes, preferably modified smectite.

In a particular embodiment, the purifying solid of the step iii) comprises:
a) a total silicon:aluminium ratio comprised between 2.0:1.0-10:1.0, preferably 5.6-1.0
b) Between 0.5-4% by weight of Mg, preferably 1.2 % by weight of Mg
c) Between 0.2-3% by weight of Fe, preferably 0.9 % by weight of Fe
d) Between 0.1-2% by weight of Ca, preferably 0.4 % by weight of Ca
e) Between 0.1-2% by weight of S, preferably 0.5 % by weight of S
f) Between 0.01-0.5 % by weight of F, preferably 0.5 % by weight of F
g) Between 0.0001-0.005 % by weight of Na, preferably 0.005 % in Na

In a particular embodiment, the purifying solid of the step iii) comprises:
a) an X-ray powder diffraction pattern, characterized in that the most intense diffraction peak appears at the 2 theta angle corresponding to 5.74° and the remaining diffraction peaks appear at 2 theta angles corresponding to 19.77°, 26.33°, 54.11°, 61.85°, 68.11° and 76.33°, ordered from greatest to lowest intensity of the associated peaks
b) a total specific area (BET) comprised between 200-800 m²/g, preferably 390 m²/g
c) a total pore volume comprised between 0.1-1 ml/g, preferably 0.5 ml/g
d) a macropore distribution where the macropore diameter is comprised between 20-2000 angstroms, preferably 20-60 angstroms, with an average diameter in terms of pore volume centered on 40 angstroms.

In a particular embodiment, the purifying solid of step iii) comprises an acidity comprised between 100-900 micromoles per gram, in a particular embodiment, this acidity is determined by thermogravimetry with n-propylamine assisted with mass spectroscopy, by a total acid center concentration.

In a particular embodiment of the present invention, the purified alkylate obtained in step iv) does not contain benzene.

### Brief Description of the Drawings

Figure 1 shows a diagram of the reaction of the present invention in the form of a flow diagram.
Figure 2 shows the resulting chromatogram crude alkylate analyzed by gas chromatography-mass spectrometry (GC-MS).
Figure 3 shows the chromatogram of the first distillation column head stream.
Figure 4 shows the chromatogram obtained by GC-FID of the second distillation column head stream.
Figure 5 shows the decrease of the sulfonation color with the content of L-DAB.
Figure 6 shows, in a simultaneous and enlarged manner, the areas of the chromatograms shown in Figures 3 and 4, corresponding to retention times longer than 50 minutes.

### Detailed Description of the Invention

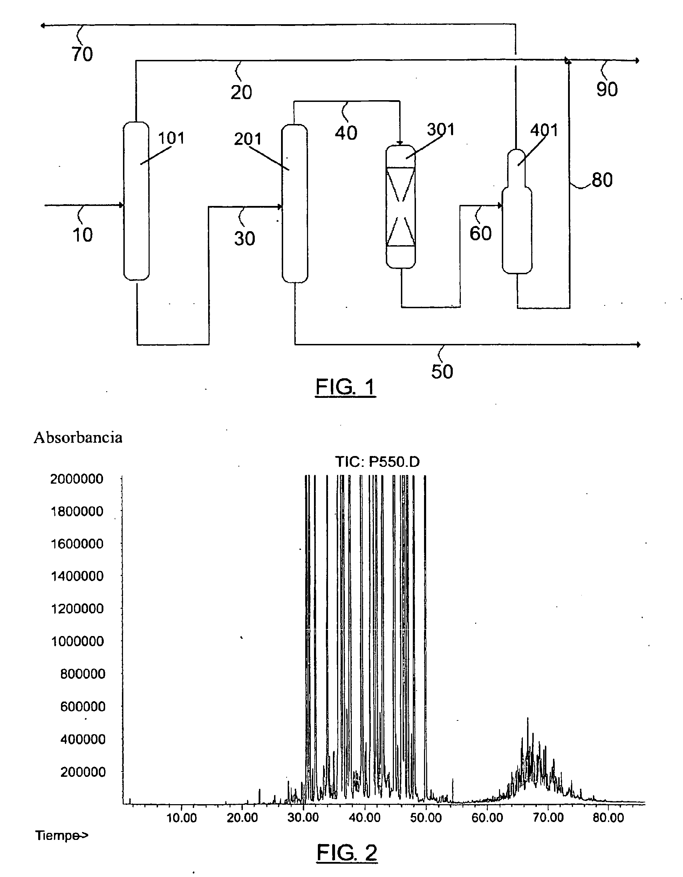

Figure 1 shows a non-limiting diagram for implementing this invention.

The crude linear alkyl aromatic compound can be obtained by means of alkylating an aromatic compound, preferably benzene, with olefins with 9 to 25 carbon atoms, preferably obtained by dehydrogenating the corresponding linear paraffins in the presence of a homogeneous catalyst, preferably HF.

Stream 10 is the crude alkylate feedstock, which is fed to the first distillation column, unit 101, in which it is separated into two fractions. The lightest fraction, approximately 75% by weight of the initial crude alkylate, is separated through the column head, stream 20, whereas the heavier second fraction emerges through he column bottom, stream 30. Stream 30 is fed to the second distillation column, unit 201. In this unit, almost all the alkylate contained in stream 30 is separated through the column head, stream 40, whereas the remaining compounds, mainly heavy alkylate, are removed through the column bottom, stream 50, and sent to be stored. Stream 40 is fed to the clay treater, unit 301, in which it is percolated through the fixed-bed containing the selected clay. The resulting alkylate, stream 60, which can contain traces of benzene associated to unwanted reactions, is fed to the benzene stripper, unit 401, in which the benzene is entrained with the aid of a nitrogen stream and/or a suitable temperature and pressure. The entrained benzene leaves the column through the head, stream 70, being able to be recirculated to the alkylation process, more specifically to the benzene column, in which the unreacted benzene is separated from the alkylate and the paraffins. The purified alkylate, stream 80, is mixed with stream 20 in order to obtain a high purity alkylate which, once sulfonated, has a very low sulfonation color.

The invention is described below, only in illustrative terms, by means of the following example, which should never be considered as limiting the scope of the present invention.

### Examples

### Example 1:

This example shows how the chromophore precursors are mainly located in the heaviest fractions of the crude alkylate, which are referred to herein as heavy alkylate. Several analyses have been carried out to evaluate said location. The first analysis was carried out on a crude alkylate obtained from a benzene alkylation process with C₁₀-C₁₃ olefins coming from the dehydrogenation of paraffins, in which the catalyst was HF. Said crude alkylate has been analyzed by gas chromatography-mass spectrometry (GC-MS). The resulting chromatogram is shown in Figure 2, as can be seen in the chromatogram of Figure 2 and in view of that set forth in the state of the art section, two main groups of compounds can be clearly distinguished in the crude alkylate. The first group of peaks, located in the retention time range from 25 to 50 minutes, corresponds to the alkylate, i.e. monoalkyl benzene in the range from C₁₀-phenyl to C₁₃-phenyl. The heavy alkylate is then located in the residence time range corresponding to times longer than 50 minutes. Within this heavy alkylate, its two subfamilies can be distinguished: dialkyl benzenes, in the range above 60 minutes, and light dialkyl benzenes, in the range from 50 to 60 minutes.

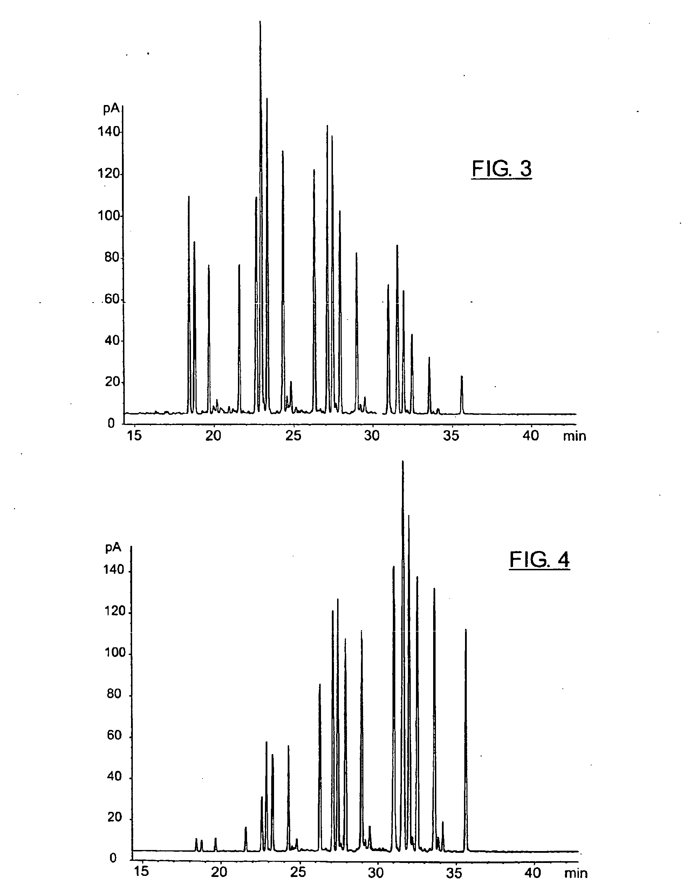

Once analyzed by GC-MS, the crude alkylate was distilled in two steps. In the first step, 75% of the starting crude alkylate was distilled in very pure alkylate form through the column head, whereas the heavy alkylate and some alkylate remained at the bottom. This heaviest fraction was then distilled again in order to separate alkylate with a certain amount of heavy species through the head and heavy alkylate through the bottom. Both the first and second distillation column head stream were analyzed by means of the UOP 698 method (GC-FID). Figure 3 shows the chromatogram of the first column head stream.

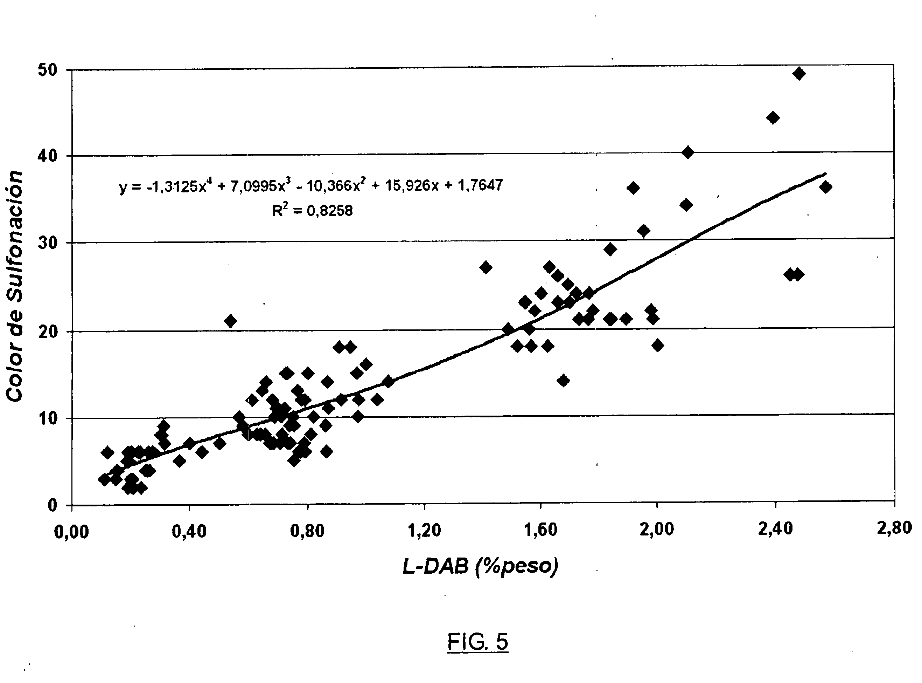

Figure 4 shows the chromatogram obtained by GC-FID of the second distillation column head stream. It can be observed that the alkylate seems to have been perfectly separated from the heavy alkylate by means of a double distillation. The only difference between chromatograms 3 and 4 seems to be due to the different relative abundance of the different homologs (different length of the alkyl chain). As can be supposed, the lightest alkylate shown in Figure 10 is richer in the lightest homologs (C₁₀-phenyl and C₁₁-phenyl), whereas the heaviest fraction of the alkylate shown in Figure 5 is richer in the heaviest homologs (C₁₂-phenyl and C₁₃-phenyl).

But when the two alkylate samples are sulfonates as established in the state of the art, it is observed that both samples have a certain color. While the sample corresponding to the lightest fraction of the alkylate has an almost non-noticeable color, the sample of the heaviest fraction of the alkylate has a quite intense color. Given that neither pure alkyl benzenes nor dialkyl benzenes (DAB) are chromophore species, the region in which the chromophores can be more easily concentrated must be analyzed. Some of the polyalkyl aromatic compounds which are responsible for the sulfonation color are eluted in the time range between light dialkyl benzenes (L-DAB) and dialkyl benzenes (C₂₆₋₃₂ DAB). Figure 5 clearly shows how the sulfonation color is correlated with the content of said light dialkyl benzenes, such that the lower the content of said compounds, the lower the sulfonation color, therefore the region of longer retention times, close to the range in which L-DAB are eluted must be focused upon in order to locate said chromophores.

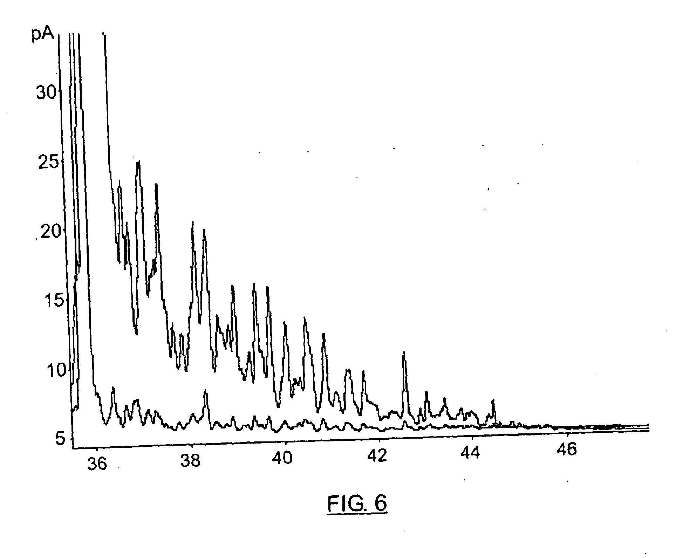

Figure 6 shows, in a simultaneous and enlarged manner, the areas of the chromatograms shown in Figures 3 and 4, corresponding to retention times longer than 50 minutes. The upper line corresponds to the chromatogram of the heaviest fraction of the alkylate (Figure 3 enlarged), whereas the lower line corresponds to the enlarged chromatogram of the lightest fraction of the alkylate (Figure 3 enlarged):
The most intense peak, appearing at a retention time of 35.8 minutes, corresponds to 2-phenyl tridecane. It can be seen that there is a considerable difference between the contents of heavy alkylate in the lightest fraction and the heaviest fraction of the alkylate. The heaviest fraction of the alkylate contains a larger amount of unwanted heavy compounds than the lightest fraction. This means that the greater sulfonation color observed upon sulfonating the heaviest fraction of the alkylate is related to a greater content of this type of heavy products, eluting in the range comprised between L-DAB and DAB, so close or even superimposed on the alkylate that it is impossible to separate it from the alkylate by means of a distillation process on an industrial scale. Therefore, a pure alkylate cannot be obtained if the operation is with a.single distillation column and if a good recovery of alkylate is desired. However, if two distillation columns are used consecutively, the chromophore species can be concentrated in the second column head stream, reducing to a great extent the amount of alkylate (25 % by weight or less) that needs to be treated with the clay.

### Example 2:

This example shows the advantages of using a selected smectite with modified acidity to eliminate color precursors from mixtures of alkyl benzenes, compared to the use of other commercial aluminosilicates.

The starting sample of linear alkylbenzenes in the range from C₁₀-phenyl to C₁₃-phenyl came from an alkylation process of olefins from the dehydrogenation of paraffins with benzene using HF as a catalyst. The benzene and the paraffins had been previously separated by distillation. The resulting crude alkylate had been distilled in two steps. In a first step, 75% by weight of the feedstock was separated through the head, providing a light fraction of alkylate with a very low content of chromophores. The bottom product, alkylate plus heavy alkylate, was fed to a second distillation column, in which it was again subjected to distillation process, obtaining all the alkylate and some impurities though the column head and heavy alkylate through the bottom.

Both alkylate samples (the lightest alkylate of the first head and the heaviest alkylate of the second head were sulfonated as defined in the state of the art. The sulfonation conditions are summarized in Table 1:

**TABLE 1**

| | |
|---|---|
| Molar SO₃ /LAB ratio | 1.10:1 |
| Reaction time (h) | 1.5 |
| Reaction temperature (°C) | 40 - 45 |
| Digestion time (h) | 1 |
| Digestion temperature (°C) | 40 - 45 |
| Hydrolysis time (h) | 0.5 |
| Hydrolysis temperature (°C) | 40 - 45 |

The composition of the alkylate, of the lightest fraction of the alkylate (first column head) and of the heaviest fraction of the alkylate (second column head) is summarized in Table 2:

**TABLE 2**

| | < C₁₀- phenyl (%weight) | C₁₀- phenyl (%weight) | C₁₁- phenyl (%weight) | C₁₂- phenyl (%weight) | C₁₃- phenyl (%weight) | > C₁₃- phenyl (%weight) | Sulfonation color (Klett scale) |
|---|---|---|---|---|---|---|---|
| Alkylate | 0.6 | 15.7 | 32.7 | 29.1 | 21.1 | 0.8 | 14 |
| Lightest alkylate | 0.8 | 20.2 | 38.7 | 27.4 | 12.4 | 0.5 | 5 |
| Heaviest alkylate | 0.1 | 2.1 | 14.7 | 34.3 | 47.1 | 1.7 | 38 |

The sulfonation color was measured by means of a Klett-Summerson colorimeter, using a 5% active ingredient solution, i.e. a solution with 5% by weight of HLAS in water, similar to the typical concentrations of HLAS (once neutralized) in commercial formulations. Sulfonation color 5 has a certain orange tone, whereas sulfonation color 38 is dark brown. Once the chromophores are concentrated in the heaviest fraction of the alkylate by means of a two-step distillation system, said fraction was subjected to percolation with different types of aluminosilicates.

Five different types of bentonites, widely used as adsorbents, were used to determine their capacity to eliminate the chromophore species from the alkylate. Said samples have been called A (acidified smectite considered in this invention), B (acidified calcium bentonite), C (acidified calcium bentonite), D (clay) and E (clay). Their chemical composition, determined by X-ray fluorescence, is summarized in Table 3 (dry base / 100% difference corresponds to losses due to ignition at 100°C):

**TABLE 3**

| compound | % by weight in solid A | % by weight in solid B | % by weight in solid C | % by weight in solid D | % by weight in solid E |
|---|---|---|---|---|---|
| Al₂O₃ | 12.26 | 15.56 | 15.61 | 15.00 | 16.5 |
| SiO₂ | 80.37 | 76.51 | 74.71 | 72.00 | 70.0 |
| SO₃ | 1.12 | ----- | ---- | ----- | ---- |
| K₂O | ----- | 1.43 | 0.92 | 1.7 | 1.2 |
| MgO | 2.04 | 1.18 | 2.04 | 1.2 | 1.5 |
| CaO | 0.57 | ----- | 0.41 | 0.3 | 0.1 |
| TiO₂ | ----- | 0.45 | 0.44 | ----- | ----- |
| Fe₂O₃ | 1.29 | 2.91 | 3.61 | 2.8 | 4.0 |
| Na (ppm) | 916 | 2236 | 1385 | 3500 | 2000 |

Their texture and acidity properties are summarized in Table 4. The acid center concentration was determined for samples A, B and C by means of a system of temperature programmed desorption (TPD) of n-propylamine, the strong centers being distinguished from the weak centers:

**TABLE 4**

| Purifying solid | A | B | C | D | E |
|---|---|---|---|---|---|
| BET area (m²/g) | 390 | 261 | 287 | 250 | 260 |
| Accumulative pore volume (ml/g) | 0.4671 | 0.4162 | 0.3597 | 0.3810 | 0.4000 |
| Strong acid centers (umol/g) | 131.9 | 120.1 | 125.8 | -------- | --------- |
| Weak acid centers (umol/g) | 178.4 | 50.3 | 99.8 | -------- | --------- |

The selected aluminosilicates were loaded in a fixed-bed located in a thermostated stainless steel reactor. Each aluminosilicate was activated by passing a hot inert gas through the bed to eliminate adsorbed water. The alkylate to be treated was then percolated through the bed in each case. The operating conditions are summarized in Table 5:

**TABLE 5**

| | |
|---|---|
| Particle diameter (um) | 30-60 |
| Bed volume (cm³) | 100 |
| Activation temperature (°C) | 120 |
| Activation LHSV (h⁻¹) | 2 |
| Activation time (h) | 12 |
| Activation fluid | N₂ |
| Purification temperature (°C) | 40-160 |
| Purification LHSV (h⁻¹) | 2 |

Once purified, each resulting alkylate sample was sulfonated as defined in Table 1. The final sulfonation color is taken as a reference to compare the performance of selected aluminosilicates. The sulfonation colors corresponding to each alkylate sample treated by each solid at the control temperatures are summarized in Table 6. The sulfonation color of the untreated alkylate is 38. The color units refer to the Klett-Summerson scale:

**TABLE 6**

| | Klett | Klett | Klett | Klett | Color |
|---|---|---|---|---|---|
| Temperature | Color | Color | Color | Color | Klett |
| (°C) | A | B | C | D | E |
| 40.0 | 3 | 12 | 18 | 15 | 17 |
| 80.0 | 2 | 9 | 11 | 6 | 16 |
| 120.0 | 5 | 11 | 11 | 17 | 21 |
| 160.0 | 15 | 15 | 25 | 28 | 36 |

As can be seen in Table 6, aluminosilicate A provides the minimum final sulfonation color in the entire tested temperature range, drastically reducing its initial value. Its effect is clearly better than that of the other tested aluminosilicates. The second best purifier, in terms of sulfonation color reduction, is aluminosilicate D. It can further be observed that the sulfonation color undergoes a first decrease upon increasing the temperature from 40°C to 80°C, followed by a color increase when the temperature is increased above 80°C in all cases.

## Claims

1. A method for purifying alkyl aromatic compounds comprising the following steps:
i) separating in a distillation column a mixture of alkyl aromatic compounds to give rise to a light fraction and a heavy fraction
ii) separating in a distillation column the heavy fraction of step i) to give rise to a light fraction and a heavy fraction
iii) eliminating the chromophore precursors from the light fraction of step ii) by means of fixed-bed percolation with a purifying solid
iv) eliminating by means of a refining column the light by-products formed in step iii)
v) mixing the purified alkylate obtained in step iv) with the lightest fraction obtained in the distillation of step i).

2. A method for purifying alkyl aromatic compounds according to claim 1, wherein the alkyl aromatic compound comprises an alkyl chain with 9-25 carbon atoms.

3. A method for purifying alkyl aromatic compounds according to any of claims 1 to 2, wherein the alkyl chains with at least 85% by weight of the alkyl aromatic compounds are linear comprise linear alkyl groups.

4. A method for purifying alkyl aromatic compounds according to any of claims 1 to 3, wherein the aromatic group forming the alkyl aromatic compounds is selected from the group consisting of: benzene, toluene and xylene.

5. A method for purifying alkyl aromatic compounds according to any of claims 1 to 4, wherein the alkyl aromatic compound comprises a maximum of 1% by weight of aromatic compounds.

6. A method for purifying alkyl aromatic compounds according to any of claims 1 to 5, wherein the alkyl aromatic compound comprises a maximum of 1% by weight of paraffins.

7. A method for purifying alkyl aromatic compounds according to any of claims 1 to 6, wherein the distillation column of step i) separates between 60-85 % by weight of the feedstock through its head.

8. A method for purifying alkyl aromatic compounds according to any of claims 1 to 7, wherein the distillation column of step i) operates at a bottom temperature comprised between 150-250°C.

9. A method for purifying alkyl aromatic compounds according to any of claims 1 to 8, wherein the distillation column of step i) operates at a head temperature comprised between 90-175 °C.

10. A method for purifying alkyl aromatic compounds according to any of claims 1 to 8, wherein the distillation column of step i) operates at a head pressure comprised between 0-1 bar.

11. A method for purifying alkyl aromatic compounds according to any of claims 1-10, wherein the distillation column of step ii) operates at a bottom temperature comprised between 175-290°C.

12. A method for purifying alkyl aromatic compounds according to any of claims 1-11, wherein the distillation column of step ii) operates at a head temperature comprised between 90 and 200°C.

13. A method for purifying alkyl aromatic compounds according to any of claims 1 to 12, wherein the distillation column of step ii) operates at a head pressure comprised between 0-1 bar.

14. A method for purifying alkyl aromatic compounds according to any of claims 1 to 13, wherein the chromophore precursor elimination step iii) is carried out at a temperature comprised between 50-150°C.

15. A method for purifying alkyl aromatic compounds according to any of claims 1 to 14, wherein the chromophore precursor elimination step iii) is carried out at a liquid space velocity comprised between 0.3-4 h⁻¹.

16. A method for purifying alkyl aromatic compounds according to any of claims 1 to 15, wherein the purifying solid of step iii) is selected from the group consisting of: zeolite, molecular sieve, silica, silica-alumina, silica gel, macroporous magnesium silicate, activated alumina, modified smectite, cellulose acetate, macroporous polystyrene gel, activated carbon and high performance organoselective polymeric membranes.

17. A method for purifying alkyl aromatic compounds according to any of claims 1 to 16, wherein the purifying solid of step iii) is a modified smectite.

18. A method for purifying alkyl aromatic compounds according to any of claims 1 to 17, wherein the purifying solid of step iii) comprises:
a) a total silicon-aluminium ratio comprised between 2.0:1.0-10:1.0
b) Between 0.5-4% by weight of Mg
c) Between 0.2-3% by weight of Fe
d) Between 0.1-2% by weight of Ca
e) Between 0.1-2% by weight of S
f) Between 0.01-0.5 % by weight of F
g) Between 0.0001-0.005 % by weight of Na

19. A method for purifying alkyl aromatic compounds according to any of claims 1 to 18, wherein the purifying solid of step iii) comprises:
a) an X-ray powder diffraction pattern, **characterized in that** the most intense diffraction peak appears at the 2 theta angle corresponding to 5.74° and the remaining diffraction peaks appear at 2 theta angles corresponding to 19.77°, 26.33°, 54.11°, 61.85°, 68.11° and 76.33°, ordered from greatest to lowest intensity of the associated peaks
b) a total specific area (BET) comprised between 200-800 m²/g
c) a total pore volume comprised between 0.1-1 ml/g
d) a macropore distribution where the macropore diameter is comprised between 20-2000 angstroms.

20. A method for purifying alkyl aromatic compounds according to any of claims 1 to 19, wherein the purifying solid of step iii) comprises an acidity of 100-900 micromoles per gram.

21. A method for purifying alkyl aromatic compounds according to any of claims 1 to 20, wherein the refining column of step iv) operates at a temperature comprised between 60-250 °C.

22. A method for purifying alkyl aromatic compounds according to any of claims 1 to 21, wherein the purified alkylate obtained in step iv) does not contain benzene.

## Patentansprüche

1. Verfahren zum Reinigen alkylaromatischer Verbindungen, das die folgenden Schritte umfasst:
i) das Auftrennen, in einer Destillationssäule, einer Mischung von alkylaromatischen Verbindungen, um zu einer leichten Fraktion und einer schweren Fraktion zu führen
ii) das Auftrennen, in einer Destillationssäule, der schweren Fraktion aus Schritt i), um zu einer leichten Fraktion und einer schweren Fraktion zu führen
iii) das Eliminieren der Chromophor-Vorläufer aus der leichten Fraktion aus Schritt ii) durch Festbettperkolation mit einem reinigenden Feststoff
iv) das Eliminieren der leichten Nebenprodukte, die in Schritt iii) gebildet worden sind, mit Hilfe einer Raffinationssäule
v) das Mischen des in Schritt iv) erhaltenen gereinigten Alkylats mit der leichtesten, bei der Destillation in Schritt i) erhaltenen Fraktion.

2. Verfahren zum Reinigen alkylaromatischer Verbindungen nach Anspruch 1, wobei die alkylaromatische Verbindung eine Alkylkette mit 9 - 25 Kohlenstoffatomen umfasst.

3. Verfahren zum Reinigen alkylaromatischer Verbindungen nach einem der Ansprüche 1 bis 2, wobei die Alkylketten, in denen mindestens 85 Gew.-% der alkylaromatischen Verbindungen linear sind, lineare Alkylgruppen umfassen.

4. Verfahren zum Reinigen alkylaromatischer Verbindungen nach einem der Ansprüche 1 - 3, wobei die aromatische Gruppe, die die alkylaromatischen Verbindungen bildet, aus der Gruppe ausgewählt ist bestehend aus: Benzol, Toluol und Xylol.

5. Verfahren zum Reinigen alkylaromatischer Verbindungen nach einem der Ansprüche 1 bis 4, wobei die alkylaromatische Verbindung maximal 1 Gew.-% aromatische Verbindungen umfasst.

6. Verfahren zum Reinigen alkylaromatischer Verbindungen nach einem der Ansprüche 1 bis 5, wobei die alkylaromatische Verbindung maximal 1 Gew.-% Paraffine umfasst.

7. Verfahren zum Reinigen alkylaromatischer Verbindungen nach einem der Ansprüche 1 bis 6, wobei die Destillationssäule von Schritt i) 60 - 85 Gew.-% des Ausgangsmaterials durch ihren Kopf abtrennt.

8. Verfahren zum Reinigen alkylaromatischer Verbindungen nach einem der Ansprüche 1 bis 7, wobei die Destillationssäule von Schritt i) bei einer Sumpftemperatur arbeitet, die zwischen 150 - 250 °C liegt.

9. Verfahren zum Reinigen alkylaromatischer Verbindungen nach einem der Ansprüche 1 bis 8, wobei die Destillationssäule von Schritt i) bei einer Kopftemperatur arbeitet, die zwischen 90 - 175 °C liegt.

10. Verfahren zum Reinigen alkylaromatischer Verbindungen nach einem der Ansprüche 1 bis 8, wobei die Destillationssäule von Schritt i) bei einem Kopfdruck arbeitet, der zwischen 0 - 1 bar liegt.

11. Verfahren zum Reinigen alkylaromatischer Verbindungen nach einem der Ansprüche 1 - 10, wobei die Destillationssäule von Schritt ii) bei einer Sumpftemperatur arbeitet, die zwischen 175 - 290 °C liegt.

12. Verfahren zum Reinigen alkylaromatischer Verbindungen nach einem der Ansprüche 1 - 11, wobei die Destillationssäule von Schritt ii) bei einer Kopftemperatur arbeitet, die zwischen 90 und 200 °C liegt.

13. Verfahren zum Reinigen alkylaromatischer Verbindungen nach einem der Ansprüche 1 bis 12, wobei die Destillationssäule von Schritt ii) bei einem Kopfdruck arbeitet, der zwischen 0 - 1 bar liegt.

14. Verfahren zum Reinigen alkylaromatischer Verbindungen nach einem der Ansprüche 1 bis 13, wobei der Chromophor-Vorläufer-Eliminationsschritt iii) bei einer Temperatur durchgeführt, die zwischen 50 150 °C liegt.

15. Verfahren zum Reinigen alkylaromatischer Verbindungen nach einem der Ansprüche 1 bis 14, wobei der Chromophor-Vorläufer-Eliminationsschritt iii) bei einer Flüssigkeitsraumgeschwindigkeit zwischen 0,3 - 4 h⁻¹ durchgeführt wird.

16. Verfahren zum Reinigen alkylaromatischer Verbindungen nach einem der Ansprüche 1 bis 15, wobei der reinigende Feststoff von Schritt iii) aus der Gruppe ausgewählt ist bestehend aus: Zeolith, Molekularsieb, Siliciumdioxid, Siliciumdioxid-Aluminiumoxid, Kieselgel, makroporösem Magnesiumsilicat, aktiviertem Aluminiumoxid, modifiziertem Smektit, Celluloseacetat, makroporösem Polystyrolgel, Aktivkohle und hochleistungsfähigen organoselektiven Polymermembranen.

17. Verfahren zum Reinigen alkylaromatischer Verbindungen nach einem der Ansprüche 1 bis 16, wobei der reinigende Feststoff von Schritt iii) ein mödifizierter Smektit ist.

18. Verfahren zum Reinigen alkylaromatischer Verbindungen nach einem der Ansprüche 1 bis 17, wobei der reinigende Feststoff von Schritt iii) Folgendes umfasst:
a) ein gesamtes Siliciumdioxid-Aluminiumoxid-Verhältnis, das zwischen 2,0: 1,0 - 10: 1,0 liegt
b) 0,5 - 4 Gew.-% Mg
c) 0,2 - 3 Gew.-% Fe
d) 0,1 - 2 Gew.-% Ca
e) 0,1 - 2 Ges.-% S
f) 0,01 - 0,5 Gew.-% F
g) 0,0001 - 0,005 Gew.-% Na.

19. Verfahren zum Reinigen alkylaromatischer Verbindungen nach einem der Ansprüche 1 bis 18, wobei der reinigende Feststoff von Schritt iii) Folgendes umfasst:
a) ein Röntgenpulver-Diffraktionsmuster, **dadurch gekennzeichnet, dass** der intensivste Peak beim 2-Theta-Winkel auftritt, der 5,74 ° entspricht und die übrigen Diffraktionspeaks bei 2 Theta-Winkeln auftreten, die 19,77 °, 26,33 °, 54,11°, 61,85°, 68,11° und 76,33° entsprechen, in der Reihenfolge von der höchsten zur geringsten Intensität der assoziierten Peaks angeordnet
b) einen gesamten spezifischen Bereich (BET), der zwischen 200 - 800 m²/g liegt
c) ein gesamtes Porenvolumen, das zwischen 0,1 - 1 ml/g liegt
d) eine Makroporenverteilung, wobei der Makroporendurchmesser zwischen 20 - 2000 Ångström liegt.

20. Verfahren zum Reinigen alkylaromatischer Verbindungen nach einem der Ansprüche 1 bis 19, wobei der reinigende Feststoff von Schritt iii) einen Säurewert von 100 - 900 Mikromol pro Gramm umfasst.

21. Verfahren zum Reinigen alkylaromatischer Verbindungen nach einem der Ansprüche 1 bis 20, wobei die Raffinationssäule von Schritt iv) bei einer Temperatur arbeitet, die zwischen 60 - 250 °C liegt.

22. Verfahren zum Reinigen alkylaromatischer Verbindungen nach einem der Ansprüche 1 bis 21, wobei das gereinigte, in Schritt iv) erhaltene Alkylat kein Benzol enthält.

## Revendications

1. Un procédé de purification de composés alkyl-aromatiques constitué des étapes suivantes :
i) séparation dans une colonne de distillation d'un mélange de composés alkyl-aromatiques pour obtenir une fraction légère et une fraction lourde.
ii) séparation dans une colonne de distillation de la fraction lourde obtenue à l'étape i) pour obtenir une fraction légère et une fraction lourde.
iii) élimination des précurseurs de chromophore de la fraction légère obtenue à l'étape ii) par une percolation à lit fixe avec un solide purifiant.
iv) élimination au moyen d'une colonne de raffinage des produits légers formés à l'étape iii)
v) mélange de l'alkylat purifié obtenu à l'étape iv) avec la fraction la plus légère obtenue dans la distillation de l'étape i).

2. Un procédé de purification de composés alkyl-aromatiques selon la revendication 1, où le composé alkyl-aromatique comprend une chaîne alkyle de 9 à 25 atomes de carbone.

3. Un procédé de purification de composés alkyl-aromatiques selon l'une quelconque des revendications 1 à 2, où les chaînes alkyles ayant une teneur en composés alkyl-aromatiques, en poids, d'au moins 85 %, sont linéaires et comprennent des groupes alkyles linéaires.

4. Un procédé de purification de composés alkyl-aromatiques selon l'une quelconque des revendications 1 à 3, où le groupe aromatique formant les composés alkyl-aromatiques est sélectionné dans le groupe constitué de : benzène, toluène et xylène.

5. Un procédé de purification de composés alkyl-aromatiques selon l'une quelconque des revendications 1 à 4, où le composé alkyl-aromatique a une teneur maximale en composés aromatiques, en poids, de 1 %.

6. Un procédé de purification de composés alkyl-aromatiques selon l'une quelconque des revendications 1 à 5, où le composé alkyl-aromatique a une teneur maximale en paraffines, en poids, de 1 %.

7. Un procédé de purification de composés alkyl-aromatiques selon l'une quelconque des revendications 1 à 6, où la colonne de distillation de l'étape i) sépare, par sa tête, de 60 à 85 % en poids du matériau.

8. Un procédé de purification de composés alkyl-aromatiques selon l'une quelconque des revendications 1 à 7, où la colonne de distillation de l'étape i) opère à une température en bas de la colonne comprise entre 150 et 250°C.

9. Un procédé de purification de composés alkyl-aromatiques selon l'une quelconque des revendications 1 à 8, où la colonne de distillation de l'étape i) opère à une température en haut de la colonne comprise entre 90 et 175°C.

10. Un procédé de purification de composés alkyl-aromatiques selon l'une quelconque des revendications 1 à 8, où la colonne de distillation de l'étape i) opère à une pression en haut de la colonne comprise entre 0 et 1 bar.

11. Un procédé de purification de composés alkyl-aromatiques selon l'une quelconque des revendications 1 à 10, où la colonne de distillation de l'étape ii) opère à une température en bas de la colonne comprise entre 175 et 290° C.

12. Un procédé de purification de composés alkyl-aromatiques selon l'une quelconque des revendications 1 à 11, où la colonne de distillation de l'étape ii) opère à une température en haut de la colonne comprise entre 90 et 200°C.

13. Un procédé de purification de composés alkyl-aromatiques selon l'une quelconque des revendications 1 à 12, où la colonne de distillation de l'étape ii) opère à une pression en haut de la colonne comprise entre 0 et 1 bar.

14. Un procédé de purification de composés alkyl-aromatiques selon l'une quelconque des revendications 1 à 13, où l'étape iii) d'élimination des précurseurs de chromophore est réalisée à une température comprise entre 50 et 150°C.

15. Un procédé de purification de composés alkyl-aromatiques selon l'une quelconque des revendications 1 à 14, où l'étape iii) d'élimination des précurseurs de chromophore est réalisée à une vitesse spatiale liquide comprise entre 0,3 et 4 h⁻¹.

16. Un procédé de purification de composés alkyl-aromatiques selon l'une quelconque des revendications 1 à 15, où le solide purifiant de l'étape iii) est sélectionné dans le groupe constitué de : zéolithe, tamis moléculaire, silice, silice alumine, gel de silice, silicate de magnésium macroporeux, alumine activée, smectite modifiée, acétate de cellulose, gel polystyrène poreux, charbon actif et membranes polymères organo-sélectives haute performance.

17. Un procédé de purification de composés alkyl-aromatiques selon l'une quelconque des revendications 1 à 16, où le solide purifiant de l'étape iii) est une smectite modifiée.

18. Un procédé de purification de composés alkyl-aromatiques selon l'une quelconque des revendications 1 à 17, où le solide purifiant de l'étape iii) comprend :
a) un rapport total silicium/aluminium compris entre 2,0/1,0 et 10/1,0
b) une teneur en Mg, en poids, de 0,5 à 4 %
c) une teneur en Fe, en poids, de 0,2 à 3 %
d) une teneur en Ca, en poids, de 0,1 à 2 %
e) une teneur en S, en poids, de 0,1 à 2 %
f) une teneur en F, en poids, de 0,01 à 0,5 %
g) une teneur en Na, en poids, de 0,0001 à 0,005 %.

19. Un procédé de purification de composés alkyl-aromatiques selon l'une quelconque des revendications 1 à 18, où le solide purifiant de l'étape iii) comprend :
a) un diagramme de diffraction X de poudre, **caractérisé en ce que** le pic de diffraction le plus fort apparaît aux angles 2-thêta correspondant à 19,77°, 26,33°, 54,11°, 61,85°, 68,11° et 76,33°, classés suivant l'intensité des pics associés, dans l'ordre décroissant.
b) une surface spécifique (BET) comprise entre 200 et 800 m²/g.
c) une porosité totale comprise entre 0,1 et 1 ml/g.
d) une distribution macroporeuse dans laquelle le diamètre du macropore est compris entre 20 et 200 angströms.

20. Un procédé de purification de composés alkyl-aromatiques selon l'une quelconque des revendications 1 à 19, où le solide purifiant de l'étape iii) comprend une acidité de 100 à 900 micromoles par gramme.

21. Un procédé de purification de composés alkyl-aromatiques selon l'une quelconque des revendications 1 à 20, où la colonne de raffinage de l'étape iv) opère à une température comprise entre 60 et 250° C.

22. Un procédé de purification de composés alkyl-aromatiques selon l'une quelconque des revendications 1 à 21, où l'alkylat purifié obtenu à l'étape iv) ne contient pas de benzène.
